# EUROPEAN PATENT APPLICATION

(11) **EP 4 607 180 A1**
(43) Date of publication of application: **27.08.2025**
(21) Application number: 23879831.8
(22) Date of filing: 18.10.2023
(51) Int. Cl.: G01N 21/3563

(54) **MEASUREMENT SYSTEM, MEAT CUTTING SYSTEM, MEASUREMENT METHOD AND PROGRAM**

(30) Priority: 19.10.2022 JP 2022167720
(71) Applicant: MAYEKAWA MFG. CO., LTD., Tokyo 135-8482 (JP)
(72) Inventor: IMAMURA Hikaru, Tokyo 135-8482 (JP); HIRAYAMA Junta, Tokyo 135-8482 (JP)
(74) Representative: Zimmermann, Tankred Klaus
(86) International application number: PCT/JP2023/037710
(87) International publication number: WO 2024/085188

(57) **Abstract**

A measurement system includes light source, light projection fiber that irradiates fat-layer-portion of meat that is measurement target with first-light from the light source, a light-receiving-fiber that is spaced apart from the light projection fiber and that receives second-light output from the fat-layer-portion of the meat when the fat-layer-portion of the meat is irradiated with the first-light, and a fat-layer-thickness-derivation-part that derives a measured thickness, which is thickness of the fat-layer-portion of the meat, based on calibration model and absorbance near absorption wavelength of fat of the second-light, the calibration model being information that associates reference thickness, which is a thickness of a fat-layer-portion of a reference meat, and an absorbance near an absorption wavelength of a fat of a light output from the fat-layer-portion of the reference meat when the fat-layer-portion of the reference meat is irradiated with light generated by the light source.

## Description

### TECHNICAL FIELD

The present invention relates to a measurement system, a meat cutting system, a measurement method and a program.

Priority is claimed on Japanese Patent Application No. 2022-167720, filed October 19, 2022, the content of which is incorporated herein by reference.

### BACKGROUND ART

An excess fat layer on a pork surface layer needs to be removed during a meat treating process. Conventionally, the excess fat layer on the pork surface layer is removed manually using a knife. For example, an operator uses a skinner to remove the fat from the pork while holding and moving it on the table with a blade fixed to the table. Since the operator does not need to hold the knife, it is safe and the work itself can be simplified. Various companies have developed various devices to simplify or automate this work. For example, in a method of recognizing fat thickness, a method of using near infrared light or impedance is known (for example, see Patent Document 1 and Patent Document 2).

### Citation List

### Patent Document

Patent Document 1: Japanese Unexamined Patent Application, First Publication No. 2007-151619
Patent Document 2: Japanese Unexamined Patent Application, First Publication No. 2010-82070

### SUMMARY OF INVENTION

### Technical Problem

The skinner cannot automate fat area recognition or meat handling.

There is a device that can automatically treat pig loin belly meat, from recognizing fat thickness to removing the fat. With this device, the fat thickness is calculated by photographing the fat exposed in a cross-section of the meat from the side with a camera and then performing image processing on the images obtained. This device cuts the fat at fixed values based on thickness data of the fat exposed in the cross-section, so if thickness of the fat changes during the process, it cannot perform cutting in accordance with the changed thickness of the fat.

In order to change the cutting depth during processing, it is necessary to perform continuous, non-destructive measurement of thickness of the fat inside the meat in real time. The non-destructive measurement method of the fat thickness described in Patent Document 1 can obtain inside information of food by impedance measurement. However, electrodes need to be securely attached to the target, making it difficult to operate them inline in a food factory.

Meanwhile, near infrared light allows excellent transparency and is already being used inline for food ingredient analysis. In Patent Document 2, near infrared light is radiated into a living body, and a transmission amount of light that is attenuated due to absorption and scattering as it passes through the body is monitored and converted into fat thickness. However, a phenomenon of light transmission is extremely complex, and there are other factors besides the presence of muscle and fat that can cause fluctuations in the amount of transmitted light, reducing measurement accuracy of the fat thickness.

The present invention is directed to providing a measurement system, a meat cutting system, a measurement method and a program that are capable of improving measurement accuracy of a fat layer thickness.

### Solution to Problem

(1) An embodiment of the present invention is a measurement system including: a light source; a light projection fiber that is configured to irradiate a fat layer portion of a meat that is a measurement target with first light from the light source; a light receiving fiber that is spaced apart from the light projection fiber and that is configured to receive second light output from the fat layer portion of the meat when the fat layer portion of the meat is irradiated with the first light; and a fat layer thickness derivation part that is configured to derive a measured thickness, which is a thickness of the fat layer portion of the meat, on the basis of a calibration model and an absorbance near an absorption wavelength of a fat of the second light, the calibration model being information that associates a reference thickness, which is a thickness of a fat layer portion of a reference meat, and an absorbance near an absorption wavelength of a fat of a light output from the fat layer portion of the reference meat when the fat layer portion of the reference meat is irradiated with light generated by the light source.
(2) According to the embodiment of the present invention, the above-mentioned measurement system further includes a spectroscope configured to generate a spectroscopic spectrum of the second light; and an absorbance derivation part configured to derive an absorbance near an absorption wavelength of a fat on the basis of the spectroscopic spectrum of the second light generated by the spectroscope, the fat layer thickness derivation part is configured to derive the measured thickness on the basis of the absorbance derived by the absorbance derivation part and the calibration model.
(3) According to the embodiment of the present invention, in the above-mentioned measurement system, the absorbance derivation part is configured to perform either or both of baseline correction and smoothing on the spectroscopic spectrum of the second light, and is configured to derive an absorbance near an absorption wavelength of a fat on the basis of the spectroscopic spectrum of the second light on which either or both of the baseline correction and the smoothing are performed.
(4) According to the embodiment of the present invention, the above-mentioned measurement system further includes a generation part configured to generate a calibration model.
(5) According to the embodiment of the present invention, in the above-mentioned measurement system, the spectroscope generates a spectroscopic spectrum of a light output from a fat layer portion of a plurality of reference meats having different thicknesses of fat layers when each of the fat layer portions of the plurality of reference meats is irradiated with light, the absorbance derivation part acquires an absorbance near an absorption wavelength of a fat on the basis of the spectroscopic spectrum of the light generated by the spectroscope, and the generation part generates the calibration model by deriving a relationship between the absorbance and the reference thicknesses of the plurality of reference meats corresponding to the spectra of the light used when the absorbance was acquired.
(6) According to the embodiment of the present invention, in the above-mentioned measurement system, the absorbance derivation part is configured to perform either or both of baseline correction and smoothing on the spectroscopic spectrum of the light, and is configured to acquire an absorbance near an absorption wavelength of a fat on the basis of the spectroscopic spectrum of the light on which either or both of the baseline correction and the smoothing have been performed.
(7) According to the embodiment of the present invention, in the above-mentioned measurement system, a range near the absorption wavelength of the fat of the second light is from 800 nm to 1100 nm.
(8) An embodiment of the present invention is a meat cutting system including: an acquisition part configured to acquire information indicating a thickness of a fat layer of a meat derived by the measurement system according to any one of the above-mentioned (1) to (7); and a controller configured to perform control of separating the meat into a fat layer and a red meat, the controller determining a thickness of the fat layer separated from the meat on the basis of the information indicating thickness of the fat layer of the meat acquired by the acquisition part.
(9) An embodiment of the present invention is a measurement method executed by a measurement system including: a light source; a light projection fiber that is configured to irradiate a fat layer portion of a meat that is a measurement target with first light from the light source; and a light receiving fiber that is spaced apart from the light projection fiber and that is configured to receive second light output from the fat layer portion of the meat when the fat layer portion of the meat is irradiated with the first light, the measurement method including: a step of irradiating the fat layer portion of the meat with the first light from the light source; a step of receiving the second light output from the fat layer portion of the meat; and a step of deriving a measured thickness, which is a thickness of the fat layer portion of the meat that is the measurement target, on the basis of a calibration model, which is information that associates a reference thickness that is a thickness of a fat layer portion of a reference meat and an absorbance near an absorption wavelength of a fat of light output from the fat layer portion of the reference meat when the fat layer portion of the reference meat is irradiated with the light generated by the light source, and an absorbance near an absorption wavelength of the fat of the second light.
(10) An embodiment of the present invention is a program configured to cause a computer of a measurement system including: a light source; a light projection fiber that is configured to irradiate a fat layer portion of a meat that is a measurement target with first light from the light source; and a light receiving fiber that is spaced apart from the light projection fiber and that is configured to receive second light output from the fat layer portion of the meat when the fat layer portion of the meat is irradiated with the first light, to perform: a step of irradiating the fat layer portion of the meat with the first light from the light source; a step of receiving the second light output from the fat layer portion of the meat; and a step of deriving a measured thickness, which is a thickness of the fat layer portion of the meat that is the measurement target, on the basis of a calibration model, which is information that associates a reference thickness that is a thickness of a fat layer portion of a reference meat to an absorbance near an absorption wavelength of a fat of light output from the fat layer portion of the reference meat when the fat layer portion of the reference meat is irradiated with the light generated by the light source, and an absorbance near an absorption wavelength of the fat of the second light.

### Advantageous Effects of Invention

According to the embodiment of the present invention, it is possible to provide a measurement system, a meat cutting system, a measurement method and a program that are capable of improving measurement accuracy of a fat layer thickness.

### BRIEF DESCRIPTION OF DRAWINGS

[FIG. 1] A view showing an example of a measurement system according to an embodiment.
[FIG. 2] A view showing an example of an operation of the measurement system according to the embodiment.
[FIG. 3] A view showing an example of an effect of the measurement system according to the embodiment.
[FIG. 4] A view showing an example of a measurement system according to Variant 1 of the embodiment.
[FIG. 5A] A view showing an example of processing of a measurement system according to Variant 1 of the embodiment.
[FIG. 5B] A view showing an example of processing of the measurement system according to Variant 1 of the embodiment.
[FIG. 6] A view showing an example of spectroscopic spectrum.
[FIG. 7] A view showing an example of an operation of the measurement system according to Variant 1 of the embodiment.
[FIG. 8] A view showing an example of a meat cutting system according to Variant 2 of the embodiment.
[FIG. 9] A schematic view showing an example of the meat cutting system according to Variant 2 of the embodiment.
[FIG. 10] A view showing an example of an information processing device according to Variant 2 of the embodiment.
[FIG. 11] A flowchart showing an example of an operation of the meat cutting system according to Variant 2 of the embodiment.
[FIG. 12] A view showing another example of a meat cutting system according to Variant 3 of the embodiment.
[FIG. 13A] A view showing an example of removal of a fat layer by a meat cutting system in the related art.
[FIG. 13B] A view showing an example of removal of a fat layer by a meat cutting system according to Variant 3 of the embodiment.

### DESCRIPTION OF EMBODIMENTS

Next, a measurement system, a meat cutting system, a measurement method and a program according to embodiments of the present invention will be described with reference to the accompanying drawings. The embodiments described below are merely examples, and the embodiments to which the present invention is applicable are not limited to the following embodiments.

Further, in all drawings to describe the embodiments, the same reference signs are used for elements having the same functions, and repeated descriptions will be omitted.

In addition, in this application, "based on XX" means "based on at least XX," and includes cases where the invention is based on other elements in addition to XX. In addition, the term "based on XX" is not limited to the direct use of XX, but also includes cases where it is based on XX that has been calculated or processed. "XX" is an arbitrary element (for example, arbitrary information).

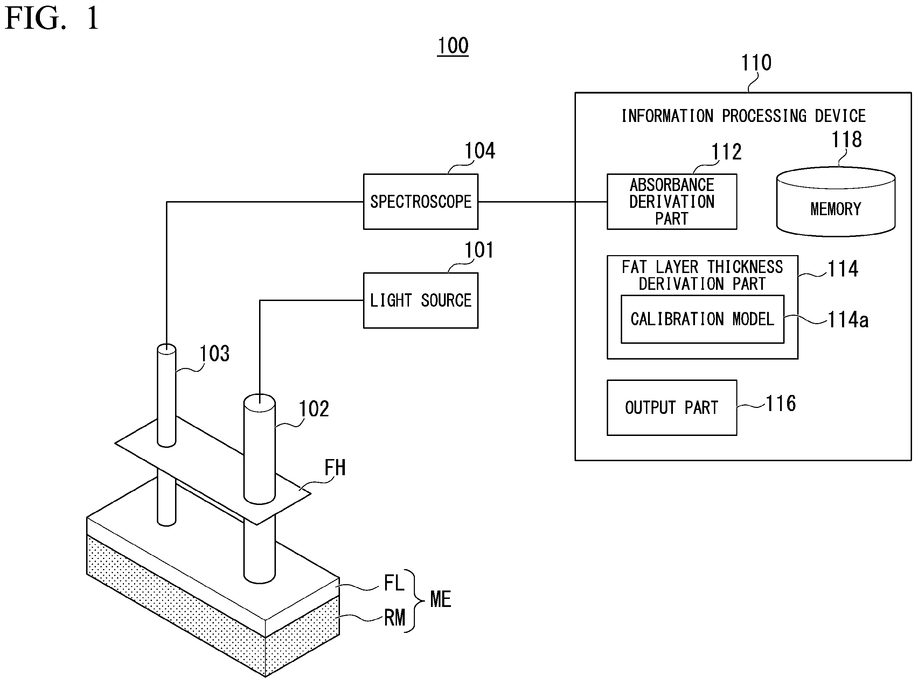

Hereinafter, a measurement system according to an embodiment of the present invention will be described with reference to the accompanying drawings. FIG. 1 is a view showing an example of a measurement system 100 according to the embodiment.

The measurement system 100 measures thickness (measured thickness, measured thickness) of a fat layer FL of a meat ME that is a measurement target. The measurement system 100 includes a light source 101, a light projection fiber 102, a light receiving fiber 103, a spectroscope 104, an information processing device 110, and a fiber holder FH.

In the example shown in FIG. 1, the light projection fiber 102 and the light receiving fiber 103 are attached to a portion of the fat layer FL of the meat ME with a distance between the light projection fiber 102 and the light receiving fiber 103. The light projection fiber 102 and the light receiving fiber 103 may be attached in contact with the portion of the fat layer FL of the meat ME. Here, the meat ME includes the fat layer FL and a red meat RM. The fiber holder FH fixes the light projection fiber 102 and the light receiving fiber 103 to the meat ME.

The light source 101 generates light (hereinafter, also referred to as "first light"). An example of the light source 101 is a halogen lamp. The halogen lamp generates electromagnetic waves in the near infrared region with a wavelength of 600 nm to 1100 nm.

The light projection fiber 102 is connected to the light source 101 and irradiates (inputs) the first light generated by the light source 101 onto the portion of the fat layer FL of the meat ME. An aspheric lens is attached to the tip of the light projection fiber 102 which is closer to the fat layer FL of the meat ME. By configuring in this way, the straightness of the first light output by the light projection fiber 102 can be maintained, so that the first light can reach the deep part of the meat ME. The light projection fiber 102 is fixed by the fiber holder FH so that the fat layer FL of the meat ME is irradiated with the first light output by the light projection fiber 102.

The light receiving fiber 103 receives light (hereinafter, also referred to as "second light") that is radiated from the light projection fiber 102 to the portion of the fat layer FL of the meat ME and is affected by at least one of diffusion, absorption and reflection inside the meat ME. The light receiving fiber 103 outputs the received second light to the spectroscope 104. The aspheric lens is mounted on the tip of the light receiving fiber 103 which is closer to the fat layer FL of the meat ME. With this configuration, the second light input to the light receiving fiber 103 can be efficiently focused, so that the light receiving fiber 103 can make the focused second light to reach the spectroscope 104. The light receiving fiber 103 is fixed by the fiber holder FH so as to be parallel to and spaced apart from the light projection fiber 102 to receive the second light. For example, the light receiving fiber 103 is fixed so as to be parallel to the light projection fiber 102 and spaced apart from it by about 10 mm to 30 mm.

The spectroscope 104 divides the second light output by the light receiving fiber 103 into separate wavelengths and measures the absorbance by receiving the light with a detector. The spectroscope 104 generates a spectroscopic spectrum by deriving the relationship between the wavelength and the absorbance of the second light.

The information processing device 110 is implemented by a device such as a personal computer, a server, a smartphone, a tablet computer, an industrial computer, or the like. The information processing device 110 includes an absorbance derivation part 112, a fat layer thickness derivation part 114, an output part 116, and a memory 118.

The memory 118 is implemented, for example, by a RAM, a ROM, a HDD, a flash memory, or a hybrid type storage device that combines two or more of these. Instead of being provided as part of the information processing device 110, a part or all of the memory 118 may be implemented as external equipment that the processor of the information processing device 110 can access via a network (not shown), such as a network attached storage (NAS), an external storage server, or the like.

The absorbance derivation part 112 acquires a spectroscopic spectrum from the spectroscope 104 and acquires the absorbance in the vicinity of the absorption wavelength of the fat (hereinafter referred to as "fat absorbance") from the acquired spectroscopic spectrum. An example near the absorption wavelength of the fat is from 800 [nm] to 1100 [nm]. In addition, an example near the absorption wavelength of the fat is from 900 [nm] to 1000 [nm].

The fat layer thickness derivation part 114 includes a calibration model 114a. The calibration model 114a is information associating thickness (reference thickness) of the portion of the fat layer FL of the reference meat ME and the absorbance in the vicinity of the absorption wavelength of the fat of the light which is output from the portion of the fat layer FL of the reference meat ME when the light generated by the light source 101 is irradiated onto the portion of the fat layer FL of the reference meat ME. An example near the absorption wavelength the fat of the light used by the fat layer thickness derivation part 114 is from 800 [nm] to 1100 [nm]. In addition, an example near the absorption wavelength of the fat of the light may be from 900 [nm] to 1000 [nm]. The calibration model 114a may be created for each distance (separating distance) between the light projection fiber 102 and the light receiving fiber 103. The calibration model 114a is created in advance. The creation of the calibration model 114a will be described below in detail

The fat layer thickness derivation part 114 acquires information indicating fat absorbance from the absorbance derivation part 112. The fat layer thickness derivation part 114 acquires thickness (reference thickness) of the portion of the fat layer FL of the reference meat ME that corresponds to the information indicating the acquired fat absorbance from the calibration model 114a, and derives thickness (measured thickness) of the portion of the fat layer FL of the meat ME, which is the measurement target.

The output part 116 acquires information indicating thickness (measured thickness) of the portion of the fat layer FL of the meat ME from the fat layer thickness derivation part 114, and outputs the acquired information indicating thickness (measured thickness) of the portion of the fat layer FL of the meat ME. For example, the output part 116 may output the information by sound or by displaying on a display unit (not shown).

All or part of the absorbance derivation part 112, the fat layer thickness derivation part 114 and the output part 116 are functional parts (hereinafter, referred to as software functional parts) that are implemented by a processor, for example, a central processing unit (CPU), executing a program stored in the memory 118.

Further, all or part of the absorbance derivation part 112, the fat layer thickness derivation part 114 and the output part 116 may be implemented by hardware such as a large scale integration (LSI), an application specific integrated circuit (ASIC), or a field-programmable gate array (FPGA), or may be implemented by a combination of a software functional part and hardware.

### (Operation of measurement system)

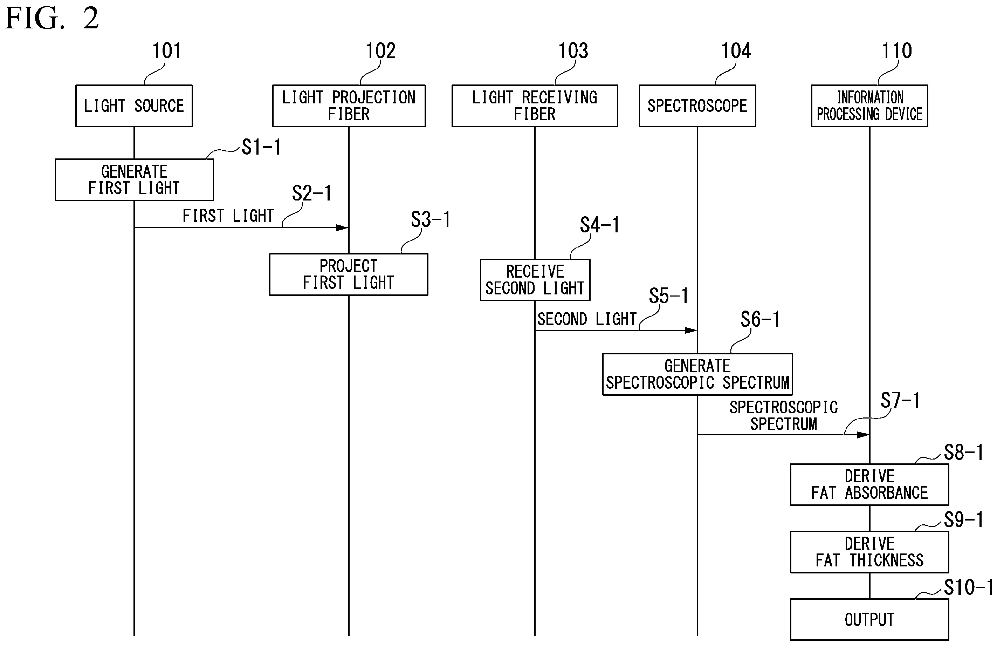

FIG. 2 is a view showing an example of an operation of the measurement system 100 according to the embodiment. Referring to FIG. 2, processing of measuring thickness (measured thickness) of the fat layer FL of the meat ME, which is a measurement target, using the measurement system 100 will be described.

### (Step S1-1)

The light source 101 generates first light.

### (Step S2-1)

The first light generated by the light source 101 is input to the light projection fiber 102.

### (Step S3-1)

The light projection fiber 102 irradiates the portion of the fat layer FL of the meat ME with the first light input from the light source 101.

### (Step S4-1)

The light receiving fiber 103 receives second light that is affected by at least one of diffusion, absorption and reflection within the meat ME.

### (Step S5-1)

The second light received by the light receiving fiber 103 is output to the spectroscope 104.

### (Step S6-1)

The spectroscope 104 divides the second light output by the light receiving fiber 103 into separate wavelengths and receives them with a detector to measure the absorbance. The spectroscope 104 generates a spectroscopic spectrum by deriving a relationship between the wavelength and the absorbance of the second light.

### (Step S7-1)

In the information processing device 110, the absorbance derivation part 112 acquires the spectroscopic spectrum from the spectroscope 104.

### (Step S8-1)

In the information processing device 110, the absorbance derivation part 112 derives fat absorbance in the vicinity of the absorption wavelength of the fat on the basis of the acquired spectroscopic spectrum.

### (Step S9-1)

In the information processing device 110, the fat layer thickness derivation part 114 acquires the information indicating the fat absorbance from the absorbance derivation part 112. The fat layer thickness derivation part 114 derives thickness (measured thickness) of the portion of the fat layer FL of the meat ME, which is a measurement target, on the basis of the calibration model 114a and the information indicating the acquired fat absorbance.

### (Step S10-1)

In the information processing device 110, the output part 116 acquires information indicating thickness (measured thickness) of the portion of the fat layer FL of the meat ME from the fat layer thickness derivation part 114, and outputs the acquired information indicating thickness (measured thickness) of the portion of the fat layer FL of the meat ME.

In the above-mentioned embodiment, as an example, although the measurement system 100 has been described as having one light receiving fiber 103, the present invention is not limited to this example. For example, the measurement system 100 may include a plurality of light receiving fibers. In this case, a calibration model is prepared for each separating distance between the light projection fiber 102 and each of the plurality of light receiving fibers. With this configuration, it is possible to simultaneously measure thickness (measured thickness) of the fat at multiple locations with different thicknesses of the fat layer FL of the meat ME.

In the above-mentioned embodiment, the measurement system 100 may not include the spectroscope 104 and the absorbance derivation part 112, and the fat layer thickness derivation part 114 may acquire the absorbance near the absorption wavelength of the fat of the second light derived outside the measurement system 100.

In the above-mentioned embodiment, the absorbance derivation part 112 may perform either or both of baseline correction and smoothing on the spectroscopic spectrum of the second light. An example of the baseline correction is spectroscopic spectrum differential processing (including second differential), standard normal variate (SNV) processing, multiplicative scatter correction (MSC) processing, mean centering, or the like. An example of the smoothing is Savitzky-Golay smoothing (SG smoothing) or the like.

For example, the absorbance derivation part 112 may remove the effects of fluctuations (baseline fluctuations) in the amount of transmitted light using common preprocessing techniques such as spectroscopic spectrum differential processing, standard normal variate (SNV) processing, multiplicative scatter correction (MSC) processing, and mean centering. In this case, the absorbance derivation part 112 derives the absorbance near the absorption wavelength of the fat on the basis of the spectroscopic spectrum of the second light that has been subjected to either or both of the baseline correction and the smoothing.

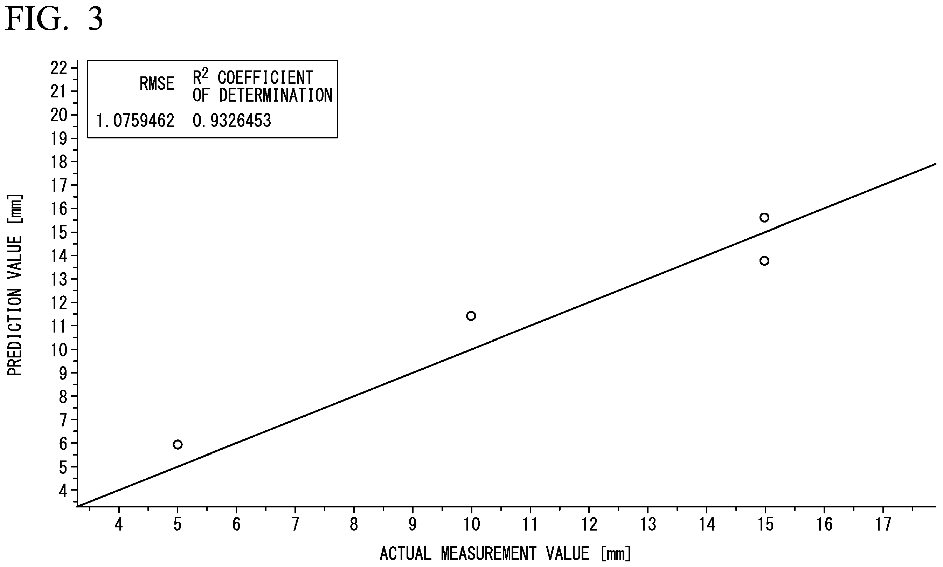

Effects of the measurement system 100 according to the embodiment will be described. FIG. 3 is a view showing an example of the effects of the measurement system 100 according to the embodiment. FIG. 3 shows a relationship between thickness (measured thickness) of the fat layer FL of the meat ME that is the measured target derived by the measurement system 100 and the actual measurement value of thickness of the fat layer FL of the meat ME that is the measurement target. In FIG. 3, a horizontal axis is an actual measurement value (mm) of thickness of the fat layer FL of the meat ME, and a vertical axis is a derivation value (prediction value) (mm) of thickness of the fat layer FL of the meat ME. A range of thickness of the fat layer FL of the meat ME that is the measurement target was 5 mm to 15 mm. The measured spectroscopic spectrum data was pre-processed (Savitzky-Golay smoothing (SG smoothing) ±20 nm, second differential) by the absorbance derivation part 112.

Prediction accuracy was evaluated using a plurality of derivation values of thickness (measured thickness) of the fat layer FL of the meat ME derived by the measurement system 100. According to FIG. 3, a root mean square error (RMSE) is 1.076, and a coefficient of determination R2 is 0.933. From the above, it can be seen that the prediction values fit the actual measurement values well.

According to the measurement system 100 of the embodiment, when the measurement system 100 irradiates a portion of the fat layer FL of the meat ME, which is the measurement target, with first light, it receives second light output from a portion of the fat layer FL that is spaced from the portion of the meat ME irradiated with the first light. After that, the measurement system 100 according to the embodiment derives thickness (measured thickness) of the portion of the fat layer FL of the meat ME on the basis of the calibration model 114a that is information associates thickness (reference thickness) of the portion of the fat layer FL of the reference meat ME and the absorbance in the vicinity of the absorption wavelength of the fat of the light output from the portion of the fat layer FL of the reference meat ME when the portion of the fat layer FL of the reference meat ME is irradiated with the light, and the absorbance in the vicinity of the absorption wavelength of the fat of the second light. For this reason, using the optical system, thickness (measured thickness) of the portion of the fat layer FL of the meat ME can be derived indirectly and non-destructively without destroying the meat ME, which is the measurement target.

Here, the vicinity of the absorption wavelength of the fat of the second light is 800 nm to 1100 nm. With this configuration, since the absorbance in the vicinity of the absorption wavelength of the fat of the second light can be acquired, thickness (measured thickness) of the portion of the fat layer FL of the meat ME can be accurately derived, compared to the case in which thickness (measured thickness) of the portion of the fat layer FL of the meat ME is derived on the basis of the calibration model 114a and the absorbance at the absorption wavelength of the fat of the second light, which has been determined in advance.

In addition, the measurement system 100 generates the spectroscopic spectrum of the second light, and derives the absorbance in the vicinity of the absorption wavelength of the fat on the basis of the generated spectroscopic spectrum of the second light. The measurement system 100 derives thickness (measured thickness) of the portion of the fat layer FL of the meat ME on the basis of the derived absorbance and the calibration model 114a. Since the absorption wavelength of the fat can be acquired by measuring the absorbance for each wavelength of the second light and capturing (detecting) the absorption peak originating from the fat from the spectroscopic spectrum of the generated second light, thickness (measured thickness) of the portion of the fat layer FL of the meat ME can be indirectly derived from the acquired absorption wavelength using the calibration model 114a. That is, thickness (measured thickness) of the portion of the fat layer FL of the meat ME can be accurately derived, compared to the case in which thickness of the portion of the fat layer FL of the meat ME is derived on the basis of the calibration model 114a and the absorbance at the previously determined absorption wavelength of the fat of the second light.

In addition, the measurement system 100 performs either or both of the baseline correction and the smoothing on the spectroscopic spectrum of the second light, and derives the absorbance in the vicinity of the absorption wavelength of the fat on the basis of the spectroscopic spectrum of the second light that has been subjected to either or both of the baseline correction and the smoothing. For this reason, compared to the case not performing either or both of the baseline correction and the smoothing on the spectroscopic spectrum of the second light, measurement error and error between workpieces can be eliminated, the estimation accuracy of thickness (measured thickness) of the portion of the fat layer FL of the meat ME can be improved.

### (Variant 1 of embodiment)

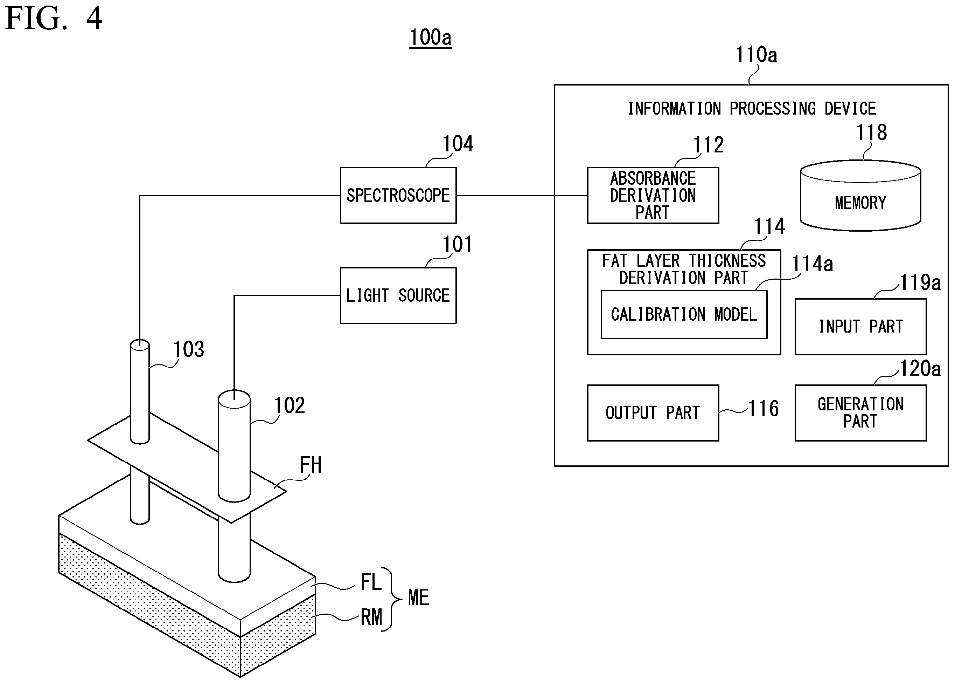

FIG. 4 is a view showing an example of a measurement system 100a according to Variant 1 of the embodiment. The measurement system 100a according to Variant 1 of the embodiment is a system including a function of creating a calibration model 114a in the measurement system 100.

The measurement system 100a measures fat thickness (measured thickness) of the meat ME. The measurement system 100a includes a light source 101, a light projection fiber 102, a light receiving fiber 103, a spectroscope 104, an information processing device 110a, and a fiber holder FH.

The information processing device 110a is implemented by a device such as a personal computer, a server, a smartphone, a tablet computer, an industrial computer, or the like. The information processing device 110a includes an absorbance derivation part 112, a fat layer thickness derivation part 114, an output part 116, a memory 118, an input part 119a, and a generation part 120a.

Processing of creating the calibration model 114a using the information processing device 110a will be described.

A portion of the fat layer FL of each of a plurality of reference meats ME with different thicknesses of the fat layer FL is irradiated with first light generated by the light source 101 via the light projection fiber 102. Thickness (reference thickness) of the fat layer FL of each of the plurality of reference meats ME is already known.

The light-receiving fiber 103 receives multiple second lights that have been affected by at least one of diffusion, absorption, and reflection inside the meat ME (inside the fat layer FL of the meat ME) after being input into the each of the portion of the fat layer FL of the plurality of meats ME from the light projection fiber 102 as described above.

The spectroscope 104 divides each of the plurality of second lights output by the light receiving fiber 103 into separate wavelengths and receives them with a detector to measure the absorbance. The spectroscope 104 generates multiple spectroscopic spectrum of the second light.

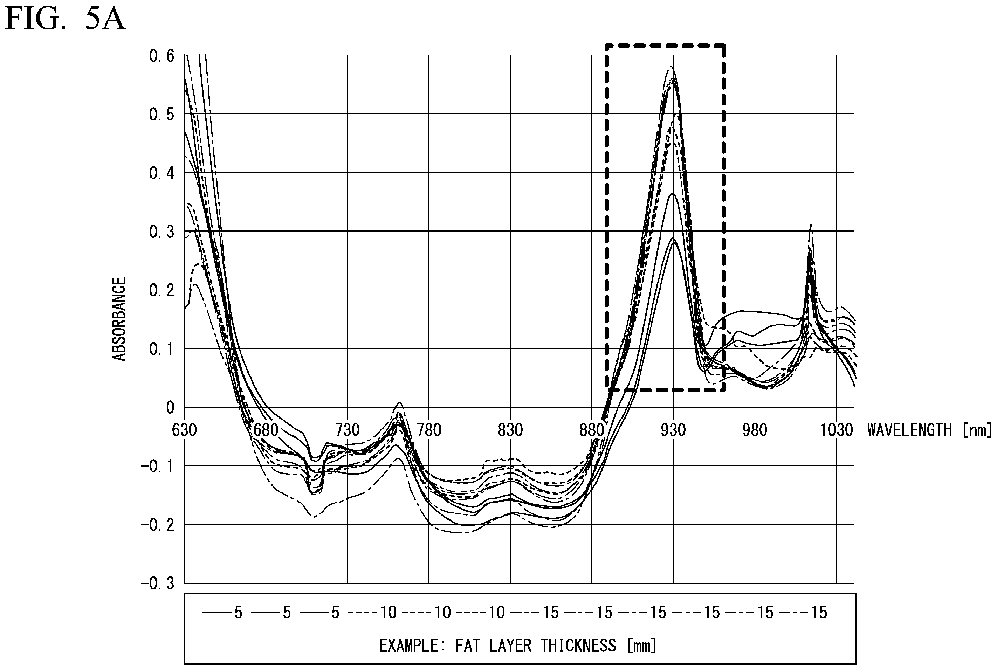

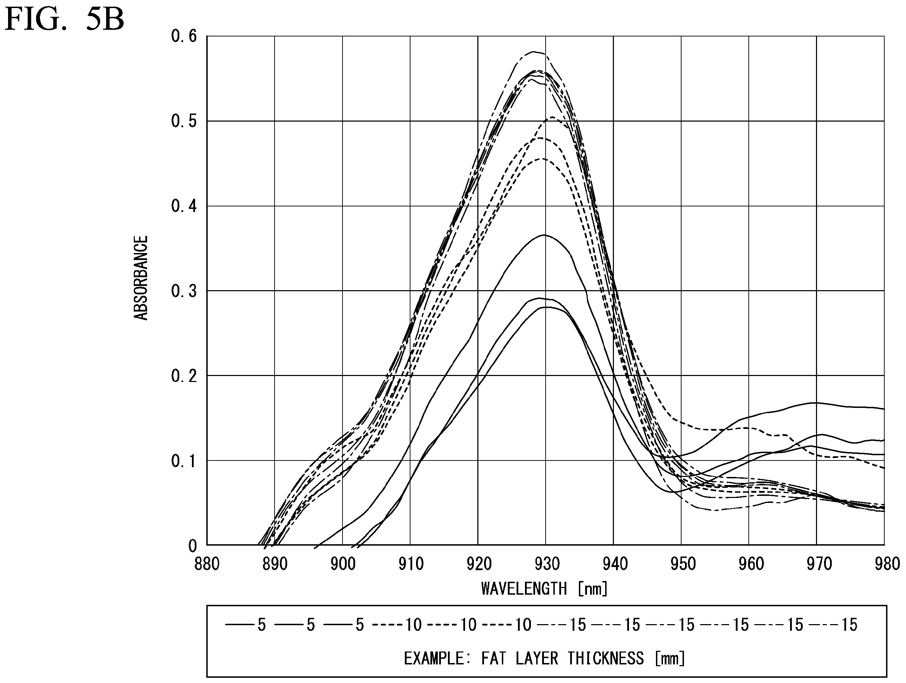

FIG. 5A and FIG. 5B are views showing an example of processing of the measurement system 100a according to Variant 1 of the embodiment. FIG. 5A is a view showing an example of a spectroscopic spectrum acquired by the absorbance derivation part 112. In FIG. 5A, a horizontal axis is a wavelength (nm), and a vertical axis is an absorbance. In FIG. 5A, as an example, thickness of the fat layer is 5 (mm), 10 (mm) and 15 (mm), and the results of measuring 5 (mm) and 10 (mm) three times and 15 (mm) six times are shown for wavelengths from 630 (nm) to 1030 (nm). According to FIG. 5A, it can be seen that, although the absorbance differs depending on thickness of the fat layer FA, similar properties are obtained.

FIG. 5B is an enlarged view of the portion with a wavelength from 880 (nm) to 980 (nm) in the spectroscopic spectrum shown in FIG. 5A. According to FIG. 5B, it can be seen that the absorbance with the wavelength of about 930 (nm) indicates a peak, and the absorbance also increases as thickness of the fat layer FA increases.

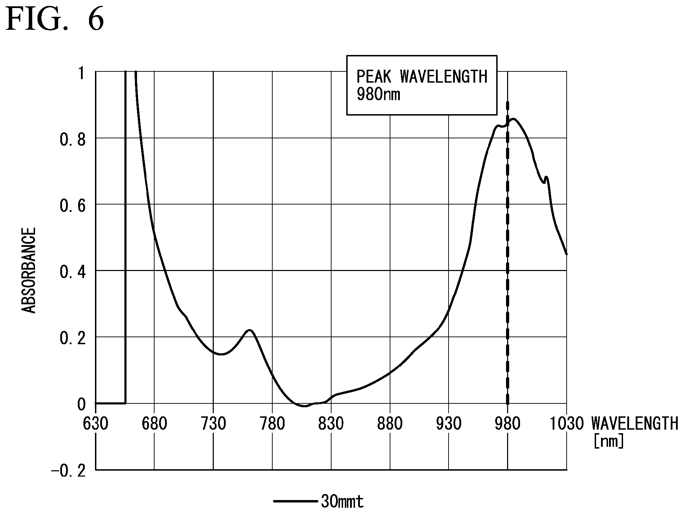

FIG. 6 is a view showing an example of the spectroscopic spectrum. FIG. 6 shows the spectroscopic spectrum of the light generated by the light source 101 being irradiated onto the portion of the red meat RM of the meat ME and being affected by at least one of diffusion, absorption and reflection within the red meat RM. In FIG. 6, a horizontal axis is a wavelength (nm), and a vertical axis is an absorbance. Further, thickness of the red meat RM is 30 (mm). According to FIG. 6, it can be seen that the absorbance with the wavelength of about 980 (nm) indicates a peak.

From FIG. 5B and FIG. 6, in the spectroscopic spectrum obtained by irradiating the fat layer FL of the meat ME with the light generated by the light source 101, an absorbance with a wavelength of about 930 (nm) indicates a peak, and in the spectroscopic spectrum obtained by irradiating the portion of the red meat RM with the light generated by the light source 101, an absorbance with a wavelength of about 980 (nm) indicates a peak. For this reason, it can be seen that it is possible to determine whether it is either the fat layer FL or the red meat RM from the spectroscopic spectrum. Referring to FIG. 4, the description will be continued.

The absorbance derivation part 112 acquires the plurality of spectra from the spectroscope 104 and acquires the fat absorbance in the vicinity of the absorption wavelength of the fat of the light from each of the acquired plurality of spectra. An example near the absorption wavelength of the fat of the light is from 800 [nm] to 1100 [nm]. In addition, an example near the absorption wavelength of the fat of the light may be from 900 [nm] to 1000 [nm].

The input part 119a inputs information. As an example, the input part 119a may have an operation part such as a keyboard, a mouse, or the like. **In** this case, the input part 119a inputs information according to an operation performed on the operation part by a user. As another example, the input part 119a may input information from external equipment. The external equipment may be, for example, a portable storage medium. Information indicating thickness (reference thickness) of the fat layer FL of each of the plurality of reference meats ME is input to the input part 119a.

The generation part 120a acquires information indicating thickness (reference thickness) of the fat layer FL of each of the plurality of reference meats ME input to the input part 119a. The generation part 120a acquires the fat absorbance corresponding to the acquired information indicating thickness (reference thickness) of the fat layer FL of each of the plurality of reference meats ME from the absorbance derivation part 112. The generation part 120a generates the calibration model 114a by deriving the relationship between thickness (reference thickness) and the fat absorbance of the fat layer FL of the reference meat ME on the basis of the plurality of pieces of information in which thickness (reference thickness) and the fat absorbance of the fat layer FL of the reference meat ME are associated.

For example, the generation part 120a generates the calibration model 114a using a general regression analysis technique such as simple regression, multiple regression, principal component (principal component analysis (PCA)) regression, partial least squares regression (partial least squares regression: PLS regression), or the like, on the basis of the plurality of pieces of information in which thickness (reference thickness) and the fat absorbance of the fat layer FL of the reference meat ME are associated. The generation part 120a outputs the created calibration model 114a to the fat layer thickness derivation part 114. The fat layer thickness derivation part 114 stores the calibration model 114a output by the generation part 120a.

All or part of the input part 119a and the generation part 120a are functional parts (hereinafter, referred to as software functional parts) that are implemented, for example, by a processor such as a CPU executing a program stored in the memory 118. Further, all or part of the input part 119a and the generation part 120a may be implemented by hardware such as LSI, ASIC, FPGA, or the like, or may be implemented by a combination of the software functional part and hardware.

### (Processing of creating calibration model)

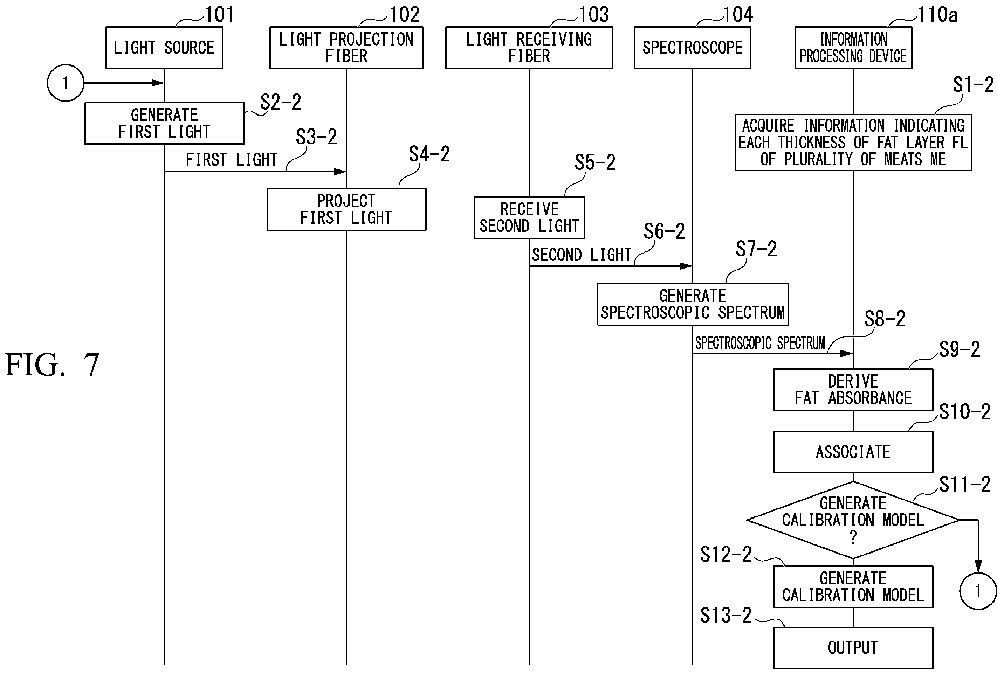

FIG. 7 is a view showing an example of processing of creating a calibration model using the measurement system 100a according to Variant 1 of the embodiment. Referring to FIG. 7, processing of creating the calibration model 114a using the measurement system 100a will be described. Since step S2-2 to S9-2 can be implemented by step S1-1 to S8-1 in FIG. 2, the description herein will be omitted.

### (Step S1-2)

In the information processing device 110a, information indicating thickness (reference thickness) of the fat layer FL of each of the plurality of reference meats ME is input to the input part 119a.

### (Step S10-2)

In the information processing device 110a, the generation part 120a acquires information indicating thickness (reference thickness) of the fat layer FL of the plurality of reference meats ME input to the input part 119a. The generation part 120a acquires the fat absorbance corresponding to the acquired information indicating thickness (reference thickness) of the fat layer FL of the plurality of reference meats ME from the absorbance derivation part 112. The generation part 120a relates thickness (reference thickness) of the fat layer FL of the reference meat ME to the fat absorbance.

### (Step S11-2)

In the information processing device 110a, the generation part 120a determines whether the calibration model 114a is created. For example, the generation part 120a determines to create the calibration model 114a when association with the fat absorbance has been completed for all of the information indicating thickness (reference thickness) of the fat layer FL of the plurality of reference meats ME acquired in step S1-2, and determines not to create the calibration model 114a when association has not been completed. The process proceeds to step S12-2 when it is determined that the generation part 120a generates the calibration model 114a, and returns to step S2-2 when it is determined that the generation part 120a does not generate the calibration model 114a.

### (Step S12-2)

In the information processing device 110a, the generation part 120a generates the calibration model 114a by deriving a relationship between thickness (reference thickness) and the fat absorbance of the fat layer FL of the reference meat ME on the basis of the plurality of pieces of information associating thickness (reference thickness) and the fat absorbance of the fat layer FL of the reference meat ME. For example, the generation part 120a generates the calibration model 114a using a method such as PLS regression analysis, principal component regression analysis, multiple regression analysis, simple regression analysis, or the like.

### (Step S13-2)

In the information processing device 110a, the generation part 120a outputs the created calibration model 114a to the fat layer thickness derivation part 114. The fat layer thickness derivation part 114 stores the calibration model 114a output by the generation part 120a.

In Variant 1 of the above-mentioned embodiment, the absorbance derivation part 112 may perform either or both of the baseline correction and the smoothing on the spectroscopic spectrum of the light, and may acquire the absorbance near the absorption wavelength of the fat, on the basis of the spectroscopic spectrum of the light on which either or both of the baseline correction and the smoothing has been performed. With this configuration, compared to the case not performing either or both of the baseline correction and the smoothing on the spectroscopic spectrum of the light, it is possible to eliminate effects such as baseline fluctuations, thereby it is possible to improve the accuracy of absorbance near the absorption wavelength of the fat.

In Variant 1 of the above-mentioned embodiment, the calibration model 114a may be created by using other device than the information processing device 110a. For example, it may be implemented by, for example, a calibration model creation device.

According to the measurement system 100a of Variant 1 of the embodiment, the measurement system 100a generates the calibration model 114a. For this reason, since the calibration model 114a can be created for each measurement system 100a, compared to the case in which the calibration model 114a is not created for each measurement system 100a, i.e., the case in which the calibration model created by the other measurement system 100a is used, derivation accuracy of thickness (measured thickness) of the fat layer FL of the meat ME that is the measurement target can be improved.

In addition, the measurement system 100a generates the calibration model 114a by generating a spectroscopic spectrum of the light output from the portion of the fat layer FL of the reference meat ME when each portion of the fat layer FL of the plurality of reference meats ME having different thicknesses of the fat layer FL are irradiated with light, by acquiring the absorbance near the absorption wavelength of the fat on the basis of the generated spectroscopic spectrum of the light, and by deriving the relationship between the absorbance and thickness (reference thickness) of the portion of the fat layer FL of the reference meat ME corresponding to the spectroscopic spectrum of the light used when the absorbance is acquired. For this reason, it is possible to derive the relationship between the absorbance and thickness (reference thickness) of the portion of the fat layer FL of the reference meat ME on the basis of the plurality of combinations of the absorbance and thickness (reference thickness) of the portion of the fat layer FL of the reference meat ME.

In addition, the measurement system 100a performs either or both of the baseline correction and the smoothing on the spectroscopic spectrum of the light, and derives the absorbance near the absorption wavelength of fat on the basis of the spectroscopic spectrum of the light that has been subjected to either or both of the baseline correction and the smoothing. For this reason, compared to the case not performing either or both of the baseline correction and the smoothing on the spectroscopic spectrum of the light, measurement errors and errors between workpieces can be eliminated, it is possible to improve the accuracy of absorbance derivation near the absorption wavelength of the fat.

### (Variant 2 of embodiment)

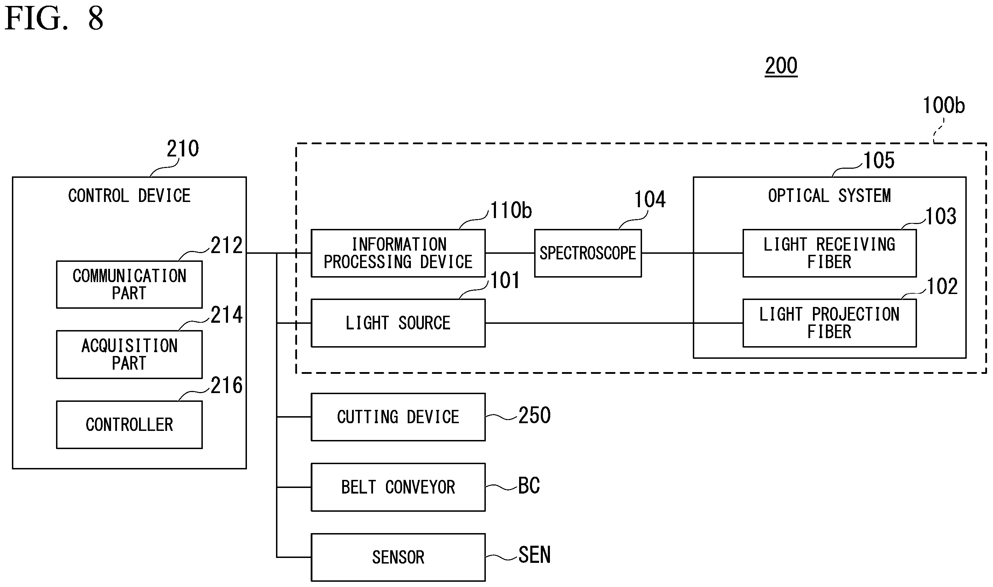

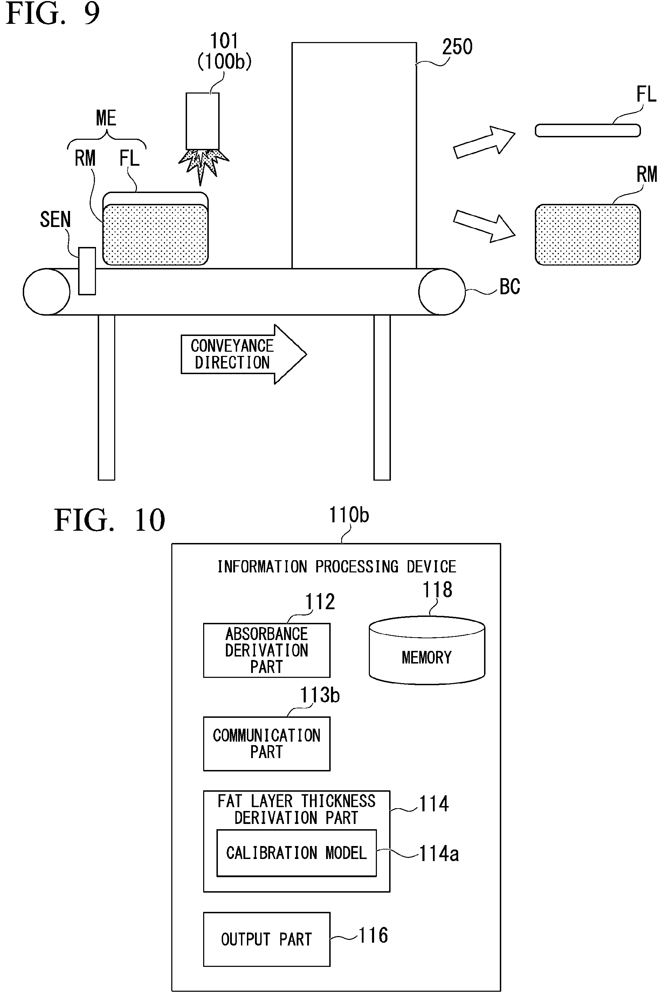

Variant 2 of the embodiment is an application of the measurement system 100 to a meat cutting system. FIG. 8 is a view showing an example of a meat cutting system 200 according to Variant 2 of the embodiment. FIG. 9 is a schematic view showing an example of the meat cutting system 200 according to Variant 2 of the embodiment.

The meat cutting system 200 is a system for separating the meat ME that is the measurement target into the fat layer FL and the red meat RM by cutting the meat ME that is the measurement target. The meat cutting system 200 includes a measurement system 100b, a control device 210, a cutting device 250, a belt conveyor BC, and a sensor SEN.

The measurement system 100b includes a light source 101, a spectroscope 104, an optical system 105, and an information processing device 110b. The optical system 105 includes a light projection fiber 102, and a light receiving fiber 103.

The belt conveyor BC includes a belt and a roller conveyor for conveyance, and conveys the meat ME that is the measurement target to the cutting device 250.

The sensor SEN is implemented by, for example, a photoelectric sensor, and installed at a predetermined position. The sensor SEN detects that the meat ME is conveyed to a predetermined position on the belt conveyor BC. When the sensor SEN detects that the meat ME has been conveyed to the predetermined position on the belt conveyor BC, it outputs detection notification information to the control device 210 to notify that the meat ME has been detected.

The control device 210 includes a communication part 212, an acquisition part 214, and a controller 216.

The communication part 212 is implemented by a communication module. The communication part 212 communicates with an external communication device such as the light source 101, the information processing device 110b, the cutting device 250, the belt conveyor BC, the sensor SEN, and the like, via a network (not shown). The communication part 212 communicates with them through a communication method such as a wired LAN or the like. The communication part 212 communicates with them through a wireless communication method such as wireless LAN, Bluetooth (Registered trademark), LTE (Registered trademark), or the like.

The communication part 212 receives detection notification information transmitted by the sensor SEN. The acquisition part 214 acquires detection notification information received by the communication part 212.

The controller 216 controls the measurement system 100b, the cutting device 250, the belt conveyor BC and the sensor SEN. The controller 216 acquires detection notification information from the acquisition part 214, and generates a measurement start trigger to cause the information processing device 110b to start measurement on the basis of the acquired detection notification information. The controller 216 transmits the created measurement start trigger from the communication part 212 to the light source 101 and the information processing device 110b.

The light source 101 receives the measurement start trigger transmitted by the control device 210 and generates light on the basis of the received measurement start trigger.

The information processing device 110b receives the measurement start trigger transmitted by the control device 210, and starts measurement of thickness (measured thickness) of the fat layer FL of the meat ME on the basis of the received measurement start trigger. The information processing device 110b measures thickness (measured thickness) of the fat layer FL of the meat ME. The information processing device 110b transmits thickness information that is the information indicating thickness (measured thickness) of the fat layer FL of the meat ME to the control device 210.

In the control device 210, the communication part 212 receives thickness information transmitted by the information processing device 110b. The acquisition part 214 acquires thickness information received by the communication part 212. The controller 216 acquires thickness information from the acquisition part 214. The controller 216 generates control information including information indicating thickness of the fat layer FL that will be separated from the meat ME on the basis of the acquired thickness information. The controller 216 outputs the created control information from the communication part 212 to the cutting device 250.

The cutting device 250 acquires the control information output by the control device 210. The cutting device 250 separates the meat ME into the fat layer FL and the red meat RM by cutting the meat ME on the basis of the information indicating thickness of the fat layer FL that will be separated from the meat ME contained in the acquired control information.

In the control device 210, all or part of the acquisition part 214 and the controller 216 are functional part (hereinafter, referred to as software functional parts) implemented by a processor such as a CPU or the like executing a program stored in a memory (not shown). Further, all or part of the acquisition part 214 and the controller 216 may be implemented by hardware such as LSI, ASIC, FPGA, or the like, or may be implemented by a combination of the software functional part and the hardware. Further, in the above-mentioned control device 210, it is also possible to configure the device without the acquisition part 214. In this case, in the control device 210, thickness information received by the communication part 212 is acquired by the controller 216, and the controller 216 generates control information including information indicating thickness of the fat layer FL that will be separated from the meat ME on the basis of the acquired thickness information. After that, the controller 216 outputs the created control information from the communication part 212 to the cutting device 250.

FIG. 10 is a view showing an example of the information processing device 110b according to Variant 2 of the embodiment. The information processing device 110b includes an absorbance derivation part 112, a communication part 113b, a fat layer thickness derivation part 114, an output part 116, and a memory 118.

The communication part 113b is implemented by a communication module. The communication part 113b communicates an external communication device such as the control device 210 or the like via a network (not shown). The communication part 113b communicates through a communication method such as a wired LAN or the like. The communication part 113b may communicate through a wireless communication method such as wireless LAN, Bluetooth (Registered trademark), LTE (Registered trademark), or the like.

The communication part 113b receives a measurement start trigger transmitted by the control device 210.

The absorbance derivation part 112 acquires the measurement start trigger received by the communication part 113b and starts measurement on the basis of the acquired measurement start trigger.

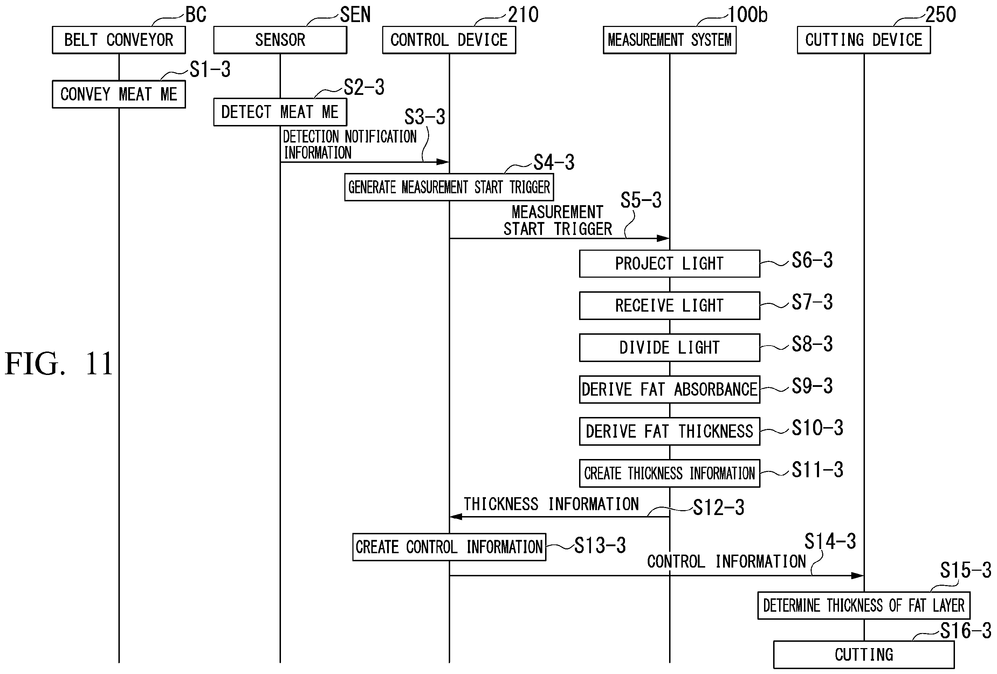

FIG. 11 is a flowchart showing an example of an operation of the meat cutting system 200 according to Variant 2 of the embodiment. Referring to FIG. 11, processing of cutting the meat ME that is the measurement target using the meat cutting system 200 will be described.

### (Step S1-3)

The belt conveyor BC conveys the meat ME to the cutting device 250 through the control of the control device 210.

### (Step S2-3)

The sensor SEN detects that the meat ME is conveyed to a predetermined position on the belt conveyor BC through the control of the control device 210.

### (Step S3-3)

The sensor SEN outputs the detection notification information to the control device 210.

### (Step S4-3)

In the control device 210, the communication part 212 receives the detection notification information transmitted by the sensor SEN. The acquisition part 214 acquires the detection notification information received by the communication part 212. The controller 216 acquires the detection notification information from the acquisition part 214 and generates the measurement start trigger on the basis of the acquired detection notification information.

### (Step S5-3)

In the control device 210, the controller 216 transmits the created measurement start trigger from the communication part 212 to the information processing device 110b and the light source 101.

### (Step S6-3)

In the measurement system 100b, the light source 101 generates first light by receiving the measurement start trigger. The first light generated by the light source 101 is input to the light projection fiber 102. The light projection fiber 102 irradiates the portion of the fat layer FL of the meat ME that is the measurement target with the first light input from the light source 101.

In the information processing device 110b, the communication part 113b receives the measurement start trigger transmitted by the control device 210. The absorbance derivation part 112 acquires the measurement start trigger received by the communication part 113b and starts measurement on the basis of the acquired measurement start trigger.

### (Step S7-3)

The light receiving fiber 103 receives the second light that is affected by at least one of diffusion, absorption and reflection within the meat ME, which is the measurement target.

### (Step S8-3)

The second light received by the light receiving fiber 103 is output to the spectroscope 104. The spectroscope 104 divides the second light output by the light receiving fiber 103 into separate wavelengths and receives them with a detector to measure the absorbance. The spectroscope 104 generates a spectroscopic spectrum by deriving a relationship between the wavelength and the absorbance of the second light.

### (Step S9-3)

In the information processing device 110b, the absorbance derivation part 112 acquires the spectroscopic spectrum from the spectroscope 104. The absorbance derivation part 112 derives the fat absorbance near the absorption wavelength of the fat on the basis of the acquired spectroscopic spectrum.

### (Step S10-3)

In the information processing device 110b, the fat layer thickness derivation part 114 acquires information indicating the fat absorbance from the absorbance derivation part 112. The fat layer thickness derivation part 114 derives thickness (measured thickness) of the portion of the fat layer FL of the meat ME on the basis of the calibration model 114a and the information indicating the acquired fat absorbance.

### (Step S11-3)

In the information processing device 110b, the output part 116 acquires information indicating thickness (measured thickness) of the portion of the fat layer FL of the meat ME from the fat layer thickness derivation part 114, and generates thickness information on the basis of the acquired information indicating thickness (measured thickness) of the portion of the fat layer FL of the meat ME.

### (Step S12-3)

In the information processing device 110b, the output part 116 transmits the created thickness information from the communication part 113b to the control device 210.

### (Step S13-3)

In the control device 210, the communication part 212 receives thickness information transmitted by the information processing device 110b. The acquisition part 214 acquires thickness information received by the communication part 212. The controller 216 acquires thickness information from the acquisition part 214. The controller 216 generates control information including information indicating thickness of the fat layer FL that will be separated from the meat ME on the basis of the acquired thickness information.

### (Step S14-3)

In the control device 210, the controller 216 outputs the created control information from the communication part 212 to the cutting device 250.

### (Step S15-3)

The cutting device 250 acquires the control information output by the control device 210. The cutting device 250 determines thickness of the fat layer FL that will be separated from the meat ME on the basis of the information indicating thickness (measured thickness) of the fat layer FL that will be separated from the meat ME contained in the acquired control information.

### (Step S16-3)

The cutting device 250 separates the meat ME into the fat layer FL and the red meat RM by cutting the meat ME through control on the basis of thickness of the fat layer FL that will be separated from the meat ME determined in step S15-3.

Hereinafter, processings from step S1-3 to step S16-3 are continuously executed.

In Variant 2 of the above-mentioned embodiment, the measurement system 100b may include the input part 119a and the generation part 120a. With this configuration, in the measurement system 100b, the calibration model 114a can be created.

In Variant 2 of the above-mentioned embodiment, as shown in FIG. 9, while the case in which the fat layer FL of the meat ME that is the measurement target is irradiated with the first light generated by the light source 101 from above the workpiece to measure thickness (measured thickness) of the fat layer FL of the meat ME has been described, it is not limited to this example. For example, the fat layer FL of the meat ME that is the measurement target may be irradiated with the first light generated by the light source 101 from a lower surface of the workpiece.

### (Variant 3 of embodiment)

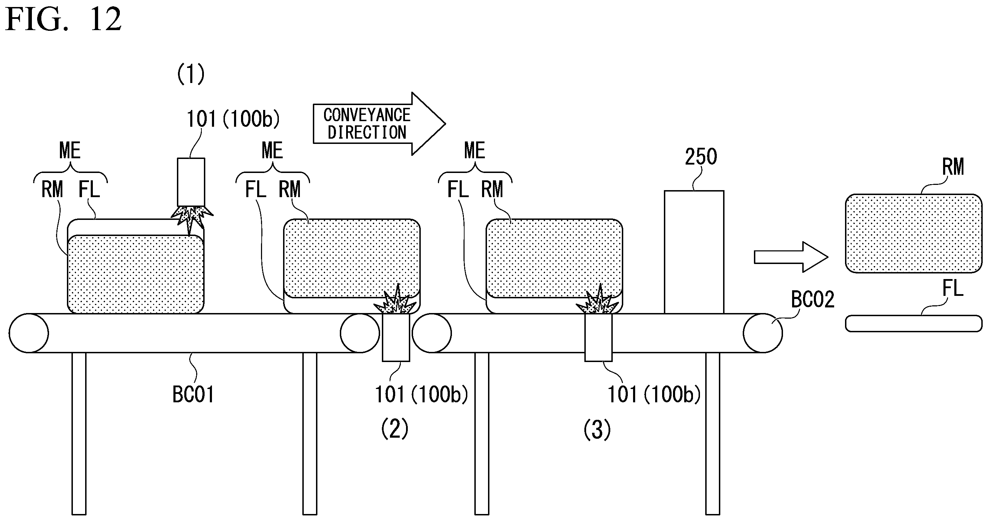

FIG. 12 is a view showing another example of the meat cutting system 200 according to Variant 3 of the embodiment. In FIG. 12, (1) shows a case in which thickness (measured thickness) of the fat layer FL is measured from above (side surface) of the workpiece as shown in FIG. 9. (2) is a place where the meat ME that is the measurement target is transferred from a belt conveyor BC01 to a belt conveyor BC02. At this place, the measurement system 100b may irradiate the fat layer FL of the meat ME with the first light generated by the light source 101 from the lower surface of the workpiece to measure thickness (measured thickness) of the fat layer FL of the meat ME. In this case, since the fat surface is the lower surface, the measurement system 100b can measure thickness (measured thickness) of the fat layer FL using a fiber installed in a fixed position. (3) is a case in which thickness (measured thickness) of the fat layer FL is measured from below the belt conveyor BC02. In this case, in the belt conveyor BC02, the fat layer FL of the meat ME may be irradiated with the first light generated by the light source 101 from the lower surface of the workpiece using a transparent belt to measure thickness (measured thickness) of the fat layer FL of the meat ME. In the belt conveyor BC02, first light emitted from the light source 101 may be irradiated onto the fat layer FL of the meat ME from below the workpiece through a gap between rollers of the roller conveyor, so that the thickness (measured thickness) of the fat layer FL of the meat ME is measured.

In Variant 3 of the above-mentioned embodiment, as an example, while the case in which the light source 101 receives the measurement start trigger transmitted by the control device 210 and generates light on the basis of the received measurement start trigger has been described, it is not limited to this example. For example, the light source 101 may be made to emit light continuously regardless of the measurement start trigger transmitted by the control device 210.

According to the meat cutting system 200 of Variant 3 of the embodiment, the meat cutting system 200 includes the acquisition part 214 configured to acquire information indicating thickness (measured thickness) of the fat layer FL of the meat ME derived by the measurement system 100b, and the controller 216 configured to perform control of separating the meat ME into the fat layer FL and the red meat RM. The controller 216 determines thickness of the fat layer FL that will be separated from the meat ME on the basis of the information indicating thickness (measured thickness) of the fat layer FL of the meat ME acquired by the acquisition part 214. For this reason, the fat layer FL can be separated from the meat ME in response to thickness of the fat layer FL of the meat ME derived by the measurement system 100b. That is, when thickness of the fat layer FL of the meat ME conveyed on the belt conveyor BC changes in a conveyance direction of the meat ME, thickness (measured thickness) of the fat layer FL of the meat ME that changes in the conveyance direction is measured by the measurement system 100b. Under the control of the controller 216 corresponding to the change in thickness (measured thickness) of the fat layer FL of the meat ME measured in this way, the cutting device 250 can separate the fat layer FL from the meat ME.

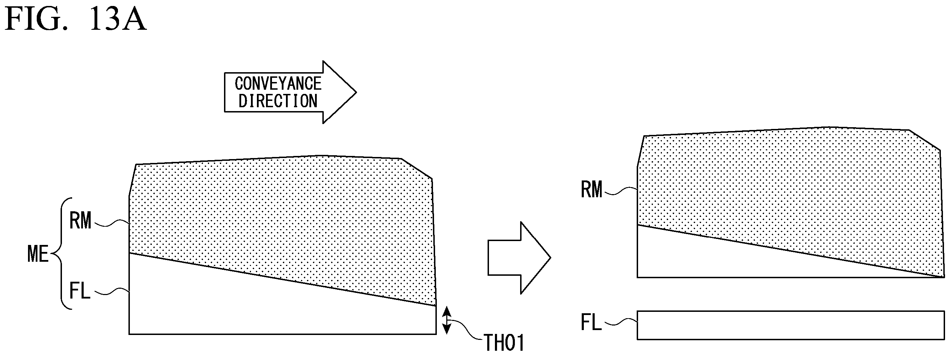

Effects of the meat cutting system according to the Variant 3 of the embodiment will be described. FIG. 13A shows an example of removal of the fat layer FL by the meat cutting system in the related art. According to FIG. 13A, thickness TH01 of the fat layer FL from the side surface of the meat ME is measured, and the meat ME is separated into the fat layer FL and the red meat RM at the measured thickness TH01 of the fat layer FL. For this reason, when thickness of the fat layer FL changes in the conveyance direction, the fat layer cannot follow the change. For example, when thickness of the fat layer FL increases in the conveyance direction, a part of the fat layer FL remains in the red meat RM, and when thickness of the fat layer FL decreases in the conveyance direction, a part of the red meat RM remains in the fat layer FL.

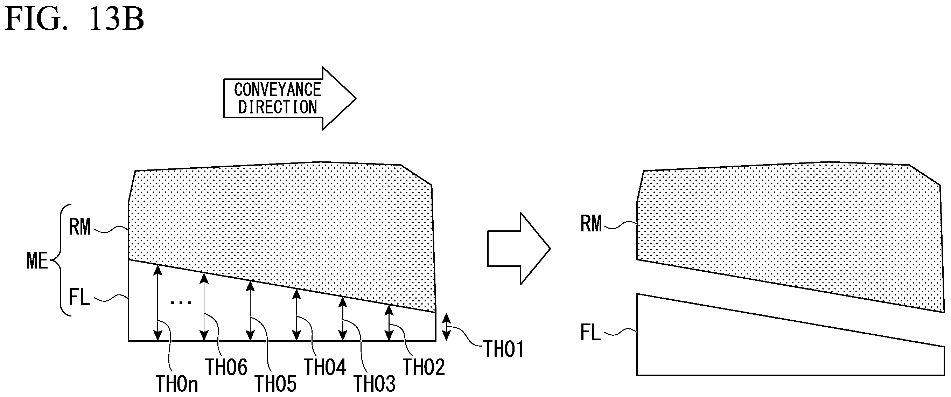

FIG. 13B shows an example of removal of the fat layer FL by the meat cutting system 200 according to Variant 3 of the embodiment. According to FIG. 13B, in addition to thickness TH01 of the fat layer FL of the side surface of the meat ME, thicknesses TH02 to TH0n (n is an integer > 1) of the fat layer FL inside the meat ME at a plurality of places are measured. The meat ME is separated into the fat layer FL and the red meat RM at the measured thicknesses TH01 to TH0n (measured thickness) at the plurality of places in the fat layer FL. That is, the controller 216 controls the cutting device 250 such that the meat M is separated into the fat layer FL and the red meat RM along the measured thicknesses TH01 to TH0n (measured thickness) at the plurality of places in the fat layer FL. In the example of removal of the fat layer FL by the meat cutting system 200 shown in FIG. 13B, when thickness of the fat layer FL changes in the conveyance direction, thickness of the fat layer FL can be measured according to such change. For this reason, when thickness of the fat layer FL increases in the conveyance direction, a part of the fat layer FL does not remain in the red meat RM, and when thickness of the fat layer FL decreases in the conveyance direction, a part of the red meat RM does not remain in the fat layer FL and the fat layer FL and the red meat RM can be separated. Further, the number of the light receiving fibers 103 included in the measurement system 100b of the meat cutting system 200 may be one or plural as described above. In the configuration in which the measurement system 100b of the meat cutting system 200 has a single light receiving fiber 103, the single light receiving fiber 103 can be used to continuously measure thickness of the fat layer FL at different positions of the meat ME in the conveyance direction of the meat ME, and to perform measurements of thicknesses TH02 to TH0n (n is an integer > 1) (measured thickness) at a plurality of positions of the fat layer FL inside the meat ME. In addition, in the configuration in which the measurement system 100b of the meat cutting system 200 includes the plurality of light receiving fibers 103, measurements of thicknesses TH02 to TH0n (n is an integer > 1) (measured thickness) at a plurality of positions of the fat layer FL inside the meat ME can be performed simultaneously. For example, as shown in FIG. 12, with respect to the meat ME conveyed on the belt conveyor, measurements of thicknesses TH02 to TH0n (n is an integer > 1) (measured thickness) can be performed at the plurality of positions of the fat layer FL inside the meat ME using the measurement system 100b of the meat cutting system 200 equipped with the one or the plurality of light receiving fibers 103.

### <Configuration example>

As a configuration example, a measurement system includes a light source, a light projection fiber that is configured to irradiate a fat layer portion of a meat that is a measurement target with first light from the light source, a light receiving fiber that is spaced apart from the light projection fiber and that is configured to receive second light output from the fat layer portion of the meat when the fat layer portion of the meat is irradiated with the first light, and a fat layer thickness derivation part that is configured to derive a measured thickness, which is thickness of the fat layer portion of the meat, on the basis of a calibration model and an absorbance near an absorption wavelength of a fat of the second light, the calibration model is information that associates a reference thickness, which is thickness of a fat layer portion of a reference meat, and an absorbance near an absorption wavelength of a fat of a light output from the fat layer portion of the reference meat when the fat layer portion of the reference meat is irradiated with light generated by the light source.

As the configuration example, the measurement system further includes a spectroscope configured to generate a spectroscopic spectrum of the second light, and an absorbance derivation part configured to derive an absorbance near an absorption wavelength of a fat on the basis of the spectroscopic spectrum of the second light generated by the spectroscope, the fat layer thickness derivation part being configured to derive the measured thickness on the basis of the absorbance derived by the absorbance derivation part and the calibration model.

As the configuration example, the absorbance derivation part is configured to perform either or both of baseline correction and smoothing on the spectroscopic spectrum of the second light, and is configured to derive an absorbance near an absorption wavelength of a fat on the basis of the spectroscopic spectrum of the second light on which either or both of the baseline correction and the smoothing are performed.

As the configuration example, the measurement system further includes a generation part configured to generate a calibration model.

As the configuration example, the spectroscope generates a spectroscopic spectrum of a light output from a fat layer portion of a plurality of reference meats having different thicknesses of fat layers when each of fat layer portions of the plurality of reference meats is irradiated with light, the absorbance derivation part acquires an absorbance near an absorption wavelength of a fat on the basis of the spectroscopic spectrum of the light generated by the spectroscope, and the generation part generates the calibration model by deriving a relationship between the absorbance and the reference thicknesses of the plurality of reference meats corresponding to the spectra of the light used when the absorbance was acquired.

As the configuration example, the absorbance derivation part performs either or both of baseline correction and smoothing on the spectroscopic spectrum of the light, and acquires an absorbance near an absorption wavelength of s fat on the basis of the spectroscopic spectrum of the light on which either or both of the baseline correction and the smoothing have been performed.

As the configuration example, a meat cutting system includes an acquisition part configured to acquire information indicating thickness of a fat layer of a meat derived by a measurement system, and a controller configured to perform control of separating the meat into a fat layer and a red meat, the controller determines thickness of the fat layer separated from the meat on the basis of the information indicating thickness of the fat layer of the meat acquired by the acquisition part. Even when an offset amount to leave the fat layer is determined, the cutting position can be calculated to cut the fat layer.

Hereinabove, although the embodiments of the present invention have been described above, these embodiments are presented as examples and are not intended to limit the scope of the invention. These embodiments can be implemented in various other forms, and various omissions, substitutions, modifications, and combinations can be made without departing from the scope of the invention. These embodiments and variants thereof are within the scope of the invention, as well as the scope of the inventions and their equivalents as described in the claims.

Further, the measurement system 100, the measurement system 100a, the measurement system 100b and the control device 210, which are described above, each have a computer. Then, each processing step of each of the above-mentioned devices is stored in the form of a program on a computer-readable recording medium, and the above-mentioned processing is performed by the computer reading and executing this program. Here, the computer-readable recording medium refers to magnetic disks, magneto-optical disks, CD-ROMs, DVD-ROMs, semiconductor memories, or the like. In addition, this computer program may be distributed to a computer via a communication line, and the computer that receives the program may execute the program.

In addition, the above-mentioned program may be intended to realize a part of the functions described above.

Further, it may be a so-called differential file (differential program) that can realize the above-mentioned functions in combination with the program already recorded in the computer system.

### REFERENCE SIGNS LIST

100, 100a, 100b Measurement system
101 Light source
102 Light projection fiber
103 Light receiving fiber
104 Spectroscope
110, 110a, 110b Information processing device
112 Absorbance derivation part
113b Communication part
114 Fat layer thickness derivation part
114a Calibration model
116 Output part
118 Memory
119a Input part
120a Generation part
200 Meat cutting system
210 Control device
212 Communication part
214 Acquisition part
216 Controller
250 Cutting device

## Claims

1. A measurement system comprising:
a light source;
a light projection fiber that is configured to irradiate a fat layer portion of a meat that is a measurement target with first light from the light source;
a light receiving fiber that is spaced apart from the light projection fiber and that is configured to receive second light output from the fat layer portion of the meat when the fat layer portion of the meat is irradiated with the first light; and
a fat layer thickness derivation part that is configured to derive a measured thickness, which is a thickness of the fat layer portion of the meat, on the basis of a calibration model and an absorbance near an absorption wavelength of a fat of the second light,
wherein the calibration model is information that associates a reference thickness, which is a thickness of a fat layer portion of a reference meat, and an absorbance near an absorption wavelength of a fat of a light output from the fat layer portion of the reference meat when the fat layer portion of the reference meat is irradiated with light generated by the light source.

2. The measurement system according to claim 1, further comprising:
a spectroscope configured to generate a spectroscopic spectrum of the second light; and
an absorbance derivation part configured to derive an absorbance near an absorption wavelength of a fat on the basis of the spectroscopic spectrum of the second light generated by the spectroscope,
wherein the fat layer thickness derivation part is configured to derive the measured thickness on the basis of the absorbance derived by the absorbance derivation part and the calibration model.

3. The measurement system according to claim 2, wherein the absorbance derivation part is configured to perform either or both of baseline correction and smoothing on the spectroscopic spectrum of the second light, and is configured to derive an absorbance near an absorption wavelength of a fat on the basis of the spectroscopic spectrum of the second light on which either or both of the baseline correction and the smoothing are performed.

4. The measurement system according to claim 2, further comprising a generation part configured to generate a calibration model.

5. The measurement system according to claim 4, wherein the spectroscope generates a spectroscopic spectrum of a light output from a fat layer portion of a plurality of reference meats having different thicknesses of fat layers when each of the fat layer portions of the plurality of reference meats is irradiated with light,
the absorbance derivation part acquires an absorbance near an absorption wavelength of a fat on the basis of the spectroscopic spectrum of the light generated by the spectroscope, and
the generation part generates the calibration model by deriving a relationship between the absorbance and the reference thicknesses of the plurality of reference meats corresponding to the spectra of the light used when the absorbance was acquired.

6. The measurement system according to claim 5, wherein the absorbance derivation part is configured to perform either or both of baseline correction and smoothing on the spectroscopic spectrum of the light, and is configured to acquire an absorbance near an absorption wavelength of a fat on the basis of the spectroscopic spectrum of the light on which either or both of the baseline correction and the smoothing have been performed.

7. The measurement system according to claim 1, wherein a range near the absorption wavelength of the fat of the second light is from 800 nm to 1100 nm.

8. A meat cutting system comprising:
an acquisition part configured to acquire information indicating a thickness of a fat layer of a meat derived by the measurement system according to any one of claims 1 to 7; and
a controller configured to perform control of separating the meat into a fat layer and a red meat,
wherein the controller determines thickness of the fat layer separated from the meat on the basis of the information indicating the thickness of the fat layer of the meat acquired by the acquisition part.

9. A measurement method executed by a measurement system comprising:
a light source;
a light projection fiber that is configured to irradiate a fat layer portion of a meat that is a measurement target with first light from the light source; and
a light receiving fiber that is spaced apart from the light projection fiber and that is configured to receive second light output from the fat layer portion of the meat when the fat layer portion of the meat is irradiated with the first light,
the measurement method comprising:
a step of irradiating the fat layer portion of the meat with the first light from the light source;
a step of receiving the second light output from the fat layer portion of the meat; and
a step of deriving a measured thickness, which is a thickness of the fat layer portion of the meat that is the measurement target, on the basis of a calibration model, which is information that associates a reference thickness that is a thickness of a fat layer portion of a reference meat and an absorbance near an absorption wavelength of a fat of light output from the fat layer portion of the reference meat when the fat layer portion of the reference meat is irradiated with the light generated by the light source, and an absorbance near an absorption wavelength of the fat of the second light.

10. A program configured to cause a computer of a measurement system comprising:
a light source;
a light projection fiber that is configured to irradiate a fat layer portion of a meat that is a measurement target with first light from the light source; and
a light receiving fiber that is spaced apart from the light projection fiber and that is configured to receive second light output from the fat layer portion of the meat when the fat layer portion of the meat is irradiated with the first light,
to perform:
a step of irradiating the fat layer portion of the meat with the first light from the light source;
a step of receiving the second light output from the fat layer portion of the meat; and
a step of deriving a measured thickness, which is a thickness of the fat layer portion of the meat that is the measurement target, on the basis of a calibration model, which is information that associates a reference thickness that is a thickness of a fat layer portion of a reference meat to an absorbance near an absorption wavelength of a fat of light output from the fat layer portion of the reference meat when the fat layer portion of the reference meat is irradiated with the light generated by the light source, and an absorbance near an absorption wavelength of the fat of the second light.
